# EUROPEAN PATENT APPLICATION

(11) **EP 4 703 480 A1**
(43) Date of publication of application: **04.03.2026**
(21) Application number: 24797096.5
(22) Date of filing: 24.04.2024
(51) Int. Cl.: C12Q 1/6806, C08F 230/02, C12Q 1/6844

(54) **SINGLE-STRANDED NUCLEIC ACID ADSORPTION INHIBITOR, NUCLEIC ACID SOLUTION, AND NUCLEIC ACID AMPLIFICATION METHOD**

(30) Priority: 28.04.2023 JP 2023074988
(71) Applicant: NOF Corporation, Shibuya-ku Tokyo 150-6019 (JP)
(72) Inventor: SUZUKI, Hirotaka, Kawasaki-shi, Kanagawa 210-0865 (JP); MATSUDA, Masaru, Kawasaki-shi, Kanagawa 210-0865 (JP)
(74) Representative: Vossius & Partner Patentanwälte Rechtsanwälte mbB
(86) International application number: PCT/JP2024/016161
(87) International publication number: WO 2024/225350

(57) **Abstract**

The present invention provides a single-stranded nucleic acid adsorption inhibitor containing one or more polymers selected from the following Group A:
[Group A]
polymer A1: a copolymer containing a constitutional unit (a) derived from 2-(meth)acryloyloxyethyl phosphorylcholine, and a constitutional unit (b) derived from C₂-C₆ alkyl (meth)acrylate having two or more hydroxy groups as substituents;
polymer A2: a copolymer containing the constitutional unit (a), and a constitutional unit (c) derived from polyethylene glycol (meth)acrylate; and
polymer A3: a copolymer containing the constitutional unit (a), and a constitutional unit (d) derived from C₁-C₆ alkyl (meth)acrylate having a phenyl group or a phenoxy group as a substituent.

## Description

### [Technical Field]

The present invention relates to a single-stranded nucleic acid adsorption inhibitor, a nucleic acid solution, and a nucleic acid amplification method.

### [Background Art]

RNA (Ribonucleic acid) is a biopolymer in which ribonucleotide monomers are linked by phosphodiester bonds.

RNA is naturally composed of ribonucleotides including the four nucleic acid bases of adenine, guanine, cytosine, and uracil, and a derivative thereof. RNA has functions such as transmission of genetic information, signal transduction, amino acid transportation in protein synthesis, metabolic control, ribozyme formation, cell organelle formation, and the like, also exists as RNA of unknown function, and constitutes genomes of some viruses.

Examples of the use of RNA in society include therapeutic targets in the medical field, constituent materials of nucleic acid pharmaceutical products, markers for detecting viruses and microorganisms in infectious disease treatment and environmental surveys, constituent materials of mRNA vaccines, and the like. Another example of use is as a constituent material of genome editing tools in genetic engineering, for example, guide RNA for site-specific nucleases of CRISPR/Cas9 (Clustered regularly interspaced short palindromic repeats/crispr associated protein 9) and the like. Yet another example is use as a raw material for health foods and food additives.

However, in all of the above-mentioned applications, nonspecific adsorption of RNA often poses a problem. That is, when manipulating, storing, or transporting RNA, RNA adsorbs to the surface of containers, microchips, and the like, particularly the surface of plastic members, and often causes a decrease in the concentration of free DNA.

In particular, when performing RNA detection, quantification, and base sequence analysis, target RNA at extremely low concentrations ranging from micromolar to attomole or less is analyzed. Furthermore, it is often difficult to obtain excess RNA, where RNA loss due to adsorption to the inner walls of containers causes a serious problem.

Problems similar to the above also occur with single-stranded RNA.

Against this background, various plastic modification technologies have been investigated to inhibit adsorption of nucleic acids to plastic containers. For example, Non Patent Literature 1 introduces a plastic tube product with a specific modification, and describes that use of this product in preparing and storing the nucleic acid concentration standards results in inhibition of nucleic acid adsorption to the container, improvement of nucleic acid yield, and improvement of the accuracy and sensitivity of applications that use the standards.

In addition, Patent Literatures 1 and 2 disclose technologies for suppressing nucleic acid adsorption by coating the surfaces of containers or biochips with a specific coating material, and by forming a layer containing a specific polymeric substance.

### [Citation List]

### [Patent Literature]

[Patent Literature 1]
   JP 2012-78365 A
[Patent Literature 2]
   JP 2006-258630 A

### [Non Patent Literature]

[Non Patent Literature 1]
Eppendorf PCR Consumables - Compatibility Guide for PCR and qPCR Cyclers

### [Summary of Invention]

### [Technical Problem]

However, the approach of improving plastics, as in Non Patent Literature 1, requires previous replacement of all members that will come into contact with nucleic acids with members that have been modified with the technology, which is inconvenient. Similarly, the techniques described in Patent Literatures 1 and 2 also require previous application of the technology to all members that will come into contact with nucleic acids, which is inconvenient.

In view of the above-mentioned problem, the present invention aims to provide a technology that achieves inhibition of adsorption of a single-stranded nucleic acid by an approach that improves the nucleic acid solution side. To be specific, the present invention aims to provide a single-stranded nucleic acid adsorption inhibitor that, when added to a nucleic acid solution, can inhibit the adsorption of the nucleic acid to containers and members, a nucleic acid solution containing the same, and a nucleic acid amplification method using the nucleic acid solution. This approach is also advantageous in that the addition concentration can be controlled according to the property of samples, which allows for a high degree of flexibility.

### [Solution to Problem]

The present invention that can achieve the above-mentioned purpose is as follows.
[1] A single-stranded nucleic acid adsorption inhibitor comprising one or more polymers selected from the following Group A:
   [Group A]
   polymer A1: a copolymer comprising a constitutional unit (a) derived from 2-(meth)acryloyloxyethyl phosphorylcholine, and a constitutional unit (b) derived from C₂-C₆ alkyl (meth)acrylate having two or more hydroxy groups as substituents;
   polymer A2: a copolymer comprising the aforementioned constitutional unit (a), and a constitutional unit (c) derived from polyethylene glycol (meth)acrylate; and
   polymer A3: a copolymer comprising the aforementioned constitutional unit (a), and a constitutional unit (d) derived from C₁-C₆ alkyl (meth)acrylate having a phenyl group or a phenoxy group as a substituent.
[2] The single-stranded nucleic acid adsorption inhibitor of the aforementioned [1], wherein the 2-(meth)acryloyloxyethylphosphorylcholine is 2-methacryloyloxyethylphosphorylcholine.
[3] The single-stranded nucleic acid adsorption inhibitor of the aforementioned [1] or [2], wherein the C₂-C₆ alkyl (meth)acrylate having two or more hydroxy groups as substituents is glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, or sorbitol mono(meth)acrylate,
   preferably glycerol mono(meth)acrylate,
   more preferably glycerol monomethacrylate.
[4] The single-stranded nucleic acid adsorption inhibitor of any one of the aforementioned [1] to [3], wherein the polyethylene glycol (meth)acrylate is
   polyethylene glycol mono(meth)acrylate, methoxypolyethylene glycol (meth)acrylate, or ethoxypolyethylene glycol (meth)acrylate,
   preferably methoxypolyethylene glycol (meth)acrylate or ethoxypolyethylene glycol (meth)acrylate,
   more preferably methoxypolyethylene glycol methacrylate or ethoxypolyethylene glycol methacrylate,
   further preferably methoxypolyethylene glycol methacrylate.
[5] The single-stranded nucleic acid adsorption inhibitor of any one of the aforementioned [1] to [4], wherein the number average molecular weight of a polyethylene glycol chain contained in the polyethylene glycol (meth)acrylate is 50 to 10,000, preferably 50 to 5,000, more preferably 50 to 1,000, further preferably 100 to 1,000.
[6] The single-stranded nucleic acid adsorption inhibitor of any one of the aforementioned [1] to [5], wherein the C₁-C₆ alkyl (meth)acrylate having a phenyl group or a phenoxy group as a substituent is
   benzyl (meth)acrylate, 1-phenylethyl (meth)acrylate, 2-phenylethyl (meth)acrylate, or phenoxyethylene glycol (meth)acrylate,
   preferably benzyl (meth)acrylate or 2-phenylethyl (meth)acrylate,
   more preferably benzyl (meth)acrylate,
   further preferably benzyl methacrylate.
[7] The single-stranded nucleic acid adsorption inhibitor of any one of the aforementioned [1] to [6], wherein the polymer A1 has a weight average molecular weight of 10,000 to 500,000, preferably 10,000 to 100,000, more preferably 10,000 to 50,000.
[8] The single-stranded nucleic acid adsorption inhibitor of any one of the aforementioned [1] to [7], wherein the polymer A2 has a weight average molecular weight of 50,000 to 1,000,000, preferably 100,000 to 500,000, more preferably 100,000 to 300,000.
[9] The single-stranded nucleic acid adsorption inhibitor of any one of the aforementioned [1] to [8], wherein the polymer A3 has a weight average molecular weight of 5,000 to 2,000,000, preferably 50,000 to 1,000,000, more preferably 100,000 to 500,000.
[10] The single-stranded nucleic acid adsorption inhibitor of any one of the aforementioned [1] to [9], wherein a proportion of the aforementioned constitutional unit (a) is 10 to 70 mol%, preferably 20 to 60 mol%, more preferably 30 to 50 mol%, relative to the total constitutional units of the polymer A1.
[11] The single-stranded nucleic acid adsorption inhibitor of any one of the aforementioned [1] to [10], wherein a proportion of the aforementioned constitutional unit (b) is 2 to 50 mol%, preferably 5 to 40 mol%, more preferably 10 to 30 mol%, relative to the total constitutional units of the polymer A1.
[12] The single-stranded nucleic acid adsorption inhibitor of any one of the aforementioned [1] to [11], wherein the polymer A1 is a copolymer consisting of the aforementioned constitutional unit (a), the aforementioned constitutional unit (b), and a constitutional unit (e) derived from other monomer different from 2-(meth)acryloyloxyethyl phosphorylcholine, C₂-C₆ alkyl (meth)acrylate having two or more hydroxy groups as substituents, polyethylene glycol (meth)acrylate, and C₁-C₆ alkyl (meth)acrylate having a phenyl group or a phenoxy group as a substituent.
[13] The single-stranded nucleic acid adsorption inhibitor of the aforementioned [12], wherein the other monomer is
   C₁-C₆ alkyl (meth)acrylate, (meth)acrylic acid, or isobornyl (meth)acrylate,
   preferably C₁-C₆ alkyl (meth)acrylate,
   more preferably butyl (meth)acrylate,
   further preferably butyl methacrylate.
[14] The single-stranded nucleic acid adsorption inhibitor of the aforementioned [12] or [13], wherein a proportion of the aforementioned constitutional unit (e) is 10 to 60 mol%, preferably 20 to 50 mol%, more preferably 30 to 45 mol%, relative to the total constitutional units of the polymer A1.
[15] The single-stranded nucleic acid adsorption inhibitor of any one of the aforementioned [1] to [14], wherein a proportion of the aforementioned constitutional unit (a) is 60 to 95 mol%, preferably 80 to 95 mol%, more preferably 85 to 95 mol%, relative to the total constitutional units of the polymer A2.
[16] The single-stranded nucleic acid adsorption inhibitor of any one of the aforementioned [1] to [15], wherein a proportion of the aforementioned constitutional unit (c) is 5 to 40 mol%, preferably 5 to 20 mol%, more preferably 5 to 15 mol%, relative to the total constitutional units of the polymer A2.
[17] The single-stranded nucleic acid adsorption inhibitor of any one of the aforementioned [1] to [16], wherein the polymer A2 is a copolymer consisting of the aforementioned constitutional unit (a) and the aforementioned constitutional unit (c).
[18] The single-stranded nucleic acid adsorption inhibitor of any one of the aforementioned [1] to [17], wherein a proportion of the aforementioned constitutional unit (a) is 60 to 95 mol%, preferably 70 to 90 mol%, more preferably 75 to 85 mol%, relative to the total constitutional units of the polymer A3.
[19] The single-stranded nucleic acid adsorption inhibitor of any one of the aforementioned [1] to [18], wherein a proportion of the aforementioned constitutional unit (d) is 5 to 40 mol%, preferably 10 to 30 mol%, more preferably 15 to 25 mol%, relative to the total constitutional units of the polymer A3.
[20] The single-stranded nucleic acid adsorption inhibitor of any one of the aforementioned [1] to [19], wherein the polymer A3 is a copolymer consisting of the constitutional unit (a) and the constitutional unit (d).
[21] The single-stranded nucleic acid adsorption inhibitor of any one of the aforementioned [1] to [20], wherein the single-stranded nucleic acid is RNA.
[22] A nucleic acid solution comprising the single-stranded nucleic acid adsorption inhibitor of any one of the aforementioned [1] to [20] and a single-stranded nucleic acid.
[23] The nucleic acid solution of the aforementioned [22], which is a nucleic acid aqueous solution.
[24] The nucleic acid solution of the aforementioned [22] or [23], wherein the single-stranded nucleic acid is RNA.
[25] A method for amplifying a nucleic acid, comprising using the nucleic acid solution of any one of the aforementioned [22] to [24].
[26] A method for inhibiting adsorption of a single-stranded nucleic acid in a nucleic acid solution to a member in contact with the aforementioned nucleic acid solution, comprising mixing the single-stranded nucleic acid adsorption inhibitor of any one of the aforementioned [1] to [20], the single-stranded nucleic acid, and a solvent.
[27] The method of the aforementioned [26], wherein the nucleic acid solution is a nucleic acid aqueous solution, and the solvent is water.
[28] The method of the aforementioned [26] or [27], wherein the single-stranded nucleic acid is RNA.

### [Advantageous Effects of Invention]

The single-stranded nucleic acid adsorption inhibitor of the present invention can inhibit free single-stranded nucleic acids present in the solution to which it is added from adsorbing to the surfaces of containers, microchips, and the like. Therefore, for example, in various tests using samples containing single-stranded nucleic acid as specimens, improvement of stability during specimen transportation, improvement of stability during storage of specimen, improvement of detection sensitivity, and the like can be expected. From another viewpoint, the improvement of stability during transportation and storage of pharmaceutical products and the like containing single-stranded nucleic acids can be expected by using the single-stranded nucleic acid adsorption inhibitor of the present invention.

### [Description of Embodiments]

The present invention is described in detail below. Respective descriptions in the present specification can be combined with each other, unless it is clear that they cannot be combined.

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

In the present specification, the "single-stranded nucleic acid" means "RNA or single-stranded DNA".

In the present specification, the "single-stranded nucleic acid adsorption inhibitor" refers to an additive used in a single-stranded nucleic acid solution to inhibit adsorption of the nucleic acid to the surface of a member that comes into contact with the solution in a container, microchip, or the like that handle the solution.

In the present specification, the "constitutional unit" means a repeat unit derived from a monomer in a polymer. Therefore, the "constitutional unit" does not include a structure not repeated in a polymer (e.g., structure derived from a polymerization initiator or the like).

In the present specification, "(meth)acrylate" basically means "acrylate" or "methacrylate". When a plurality of (meth)acrylate may be present, the "(meth)acrylate" means "acrylate and/or methacrylate". Other terms similar to "(meth)acrylate" also mean the same as the "(meth)acrylate".

In the present specification, the "C₂-C₆ alkyl (meth)acrylate" means "alkyl (meth)acrylate in which the alkyl group has 2 to 6 carbon atoms". Other terms similar to "C₂-C₆ alkyl (meth)acrylate" also mean the same as the "C₂-C₆ alkyl (meth)acrylate".

In the present specification, the "C₂-C₆ alkyl (meth)acrylate having two or more hydroxy groups as substituents" means "C₂-C₆ alkyl (meth)acrylate in which the alkyl group has two or more hydroxy groups as substituents".

In the present specification, the "polyethylene glycol (meth)acrylate" means "ester of one polyethylene glycol and one (meth)acrylic acid". The end opposite the (meth)acrylate end of the "polyethylene glycol (meth)acrylate" may be a hydroxy group (e.g., polyethylene glycol mono(meth)acrylate), methoxy group, etc. (e.g., methoxypolyethylene glycol (meth)acrylate).

In the present specification, the "polyethylene glycol mono(meth)acrylate" means "monoester of polyethylene glycol and (meth)acrylic acid, in which the end on the opposite side of the (meth)acrylate end is a hydroxy group.

In the present specification, the "methoxypolyethylene glycol (meth)acrylate" means "a compound in which the hydroxy group at the polyethylene glycol mono(meth)acrylate end is replaced with a methoxy group". Other terms similar to "methoxypolyethylene glycol (meth)acrylate" also mean the same as the "methoxypolyethylene glycol (meth)acrylate".

In the present specification, the "C₁-C₆ alkyl (meth)acrylate having a phenyl group or a phenoxy group as a substituent" means "C₁-C₆ alkyl (meth)acrylate in which the alkyl group has a phenyl group or a phenoxy group as a substituent". The aforementioned number of carbon atoms is the number of carbon atoms in the alkyl group, and does not include the number of carbon atoms in the substituent thereof (phenyl group or phenoxy group).

### [Single-stranded nucleic acid adsorption inhibitor]

The single-stranded nucleic acid adsorption inhibitor of the present invention contains one or more polymers selected from the following Group A:
[Group A]
polymer A1: a copolymer containing a constitutional unit (a) derived from 2-(meth)acryloyloxyethyl phosphorylcholine (hereinafter sometimes referred to as "monomer a"), and a constitutional unit (b) derived from C₂-C₆ alkyl (meth)acrylate having two or more hydroxy groups as substituents (hereinafter sometimes referred to as "monomer b");
polymer A2: a copolymer containing the aforementioned constitutional unit (a), and a constitutional unit (c) derived from polyethylene glycol (meth)acrylate (hereinafter sometimes referred to as "monomer c"); and
polymer A3: a copolymer containing the aforementioned constitutional unit (a), and a constitutional unit (d) derived from C₁-C₆ alkyl (meth)acrylate having a phenyl group or a phenoxy group as a substituent (hereinafter sometimes referred to as "monomer d").

Monomer a forming the constitutional unit (a) contained in polymers A1 to A3 (hereinafter sometimes referred to as "the polymer of the present invention") is 2-methacryloyloxyethyl phosphorylcholine or 2-acryloyloxyethyl phosphorylcholine, and 2-methacryloyloxyethyl phosphorylcholine is preferred from the viewpoint of storage stability of the polymer of the present invention.

Only one kind of monomer a may be used, or two kinds thereof may be used in combination. As monomer a, a commercially available product can be used, or it may be produced by a known method.

Examples of the monomer b forming the constitutional unit (b) contained in polymer A1 include glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono (meth)acrylate, and the like. From the viewpoints of raw material availability and single-stranded nucleic acid adsorption inhibitor effect, glycerol mono(meth)acrylate is preferred and glycerol monomethacrylate is more preferred from among these. In the present specification, "glycerol mono(meth)acrylate" means "monoester of glycerol and (meth)acrylic acid". Other terms similar to "glycerol mono(meth)acrylate" also mean the same as the "glycerol mono(meth)acrylate".

Only one kind of monomer b may be used, or two or more kinds thereof may be used in combination. As monomer b, a commercially available product can be used, or it may be produced by a known method.

From the viewpoint of the single-stranded nucleic acid adsorption inhibitory effect, the proportion of constitutional unit (a) relative to the total constitutional units of polymer A1 is preferably 10 to 70 mol%, more preferably 20 to 60 mol%, further preferably 30 to 50 mol%.

From the viewpoint of the single-stranded nucleic acid adsorption inhibitory effect, the proportion of constitutional unit (b) relative to the total constitutional units of polymer A1 is preferably 2 to 50 mol%, more preferably 5 to 40 mol%, further preferably 10 to 30 mol%.

While the weight average molecular weight of polymer A1 is not particularly limited, it is preferably 10,000 to 500,000, more preferably 10,000 to 100,000, further preferably 10,000 to 50,000, from the viewpoint of the single-stranded nucleic acid adsorption inhibitory effect. The weight average molecular weight can be determined based on polyethylene glycol standard by gel filtration chromatography using, for example, EcoSEC system (manufactured by Tosoh Corporation) or the like.

Examples of the monomer c forming the constitutional unit (c) contained in polymer A2 include polyethylene glycol mono(meth)acrylate, methoxypolyethylene glycol (meth)acrylate, ethoxypolyethylene glycol (meth)acrylate, and the like. From among these, methoxypolyethylene glycol (meth)acrylate and ethoxypolyethylene glycol (meth)acrylate are preferred, methoxypolyethylene glycol methacrylate and ethoxypolyethylene glycol methacrylate are more preferred, and methoxypolyethylene glycol methacrylate is further preferred, from the viewpoint of storage stability of the polymer of the present invention.

The number average molecular weight of the polyethylene glycol chain contained in monomer c is preferably 50 to 10,000, more preferably 50 to 5,000, further preferably 50 to 1,000, particularly preferably 100 to 1,000, from the viewpoints of raw material availability and single-stranded nucleic acid adsorption inhibitory effect. The number average molecular weight of the polyethylene glycol chain can be determined based on the hydroxyl value calculated, for example, by the method described in JIS K 1557, or can be determined based on polyethylene glycol standard by, for example, gel filtration chromatography.

Only one kind of monomer c may be used, or two or more kinds thereof may be used in combination. As monomer c, a commercially available product can be used, or it may be produced by a known method.

From the viewpoint of the single-stranded nucleic acid adsorption inhibitory effect, the proportion of constitutional unit (a) relative to the total constitutional units of polymer A2 is preferably 60 to 95 mol%, more preferably 80 to 95 mol%, further preferably 85 to 95 mol%.

From the viewpoint of the single-stranded nucleic acid adsorption inhibitory effect, the proportion of constitutional unit (c) relative to the total constitutional units of polymer A2 is preferably 5 to 40 mol%, more preferably 5 to 20 mol%, further preferably 5 to 15 mol%.

While the weight average molecular weight of polymer A2 is not particularly limited, it is preferably 50,000 to 1,000,000, more preferably 100,000 to 500,000, further preferably 100,000 to 300,000.

Examples of the monomer d forming the constitutional unit (d) contained in polymer A3 include benzyl (meth)acrylate, 1-phenylethyl (meth)acrylate, 2-phenylethyl (meth)acrylate, and phenoxyethylene glycol (meth)acrylate. From among these, benzyl (meth)acrylate and 2-phenylethyl (meth)acrylate are preferred, and benzyl (meth)acrylate is more preferred, from the viewpoints of raw material availability and single-stranded nucleic acid adsorption inhibitory effect. In addition, benzyl methacrylate is further preferred from the viewpoint of storage stability of polymer A3.

Only one kind of monomer d may be used, or two or more kinds thereof may be used in combination. As monomer d, a commercially available product can be used, or it may be produced by a known method.

From the viewpoint of the single-stranded nucleic acid adsorption inhibitory effect, the proportion of constitutional unit (a) relative to the total constitutional units of polymer A3 is preferably 60 to 95 mol%, more preferably 70 to 90 mol%, further preferably 75 to 85 mol%.

From the viewpoint of the single-stranded nucleic acid adsorption inhibitory effect, the proportion of constitutional unit (d) relative to the total constitutional units of polymer A3 is preferably 5 to 40 mol%, more preferably 10 to 30 mol%, further preferably 15 to 25 mol%.

While the weight average molecular weight of polymer A3 is not particularly limited, it is preferably 5,000 to 2,000,000, more preferably 50,000 to 1,000,000, further preferably 100,000 to 500,000.

Among the polymers of the present invention, a copolymer containing constitutional unit (b) (e.g., copolymer containing constitutional unit (a) to constitutional unit (c), copolymer containing constitutional unit (a), constitutional unit (b) and constitutional unit (d), and copolymer containing constitutional unit (a) to constitutional unit (d)) are classified as polymer A1, not polymer A2 or A3.

The proportion of constitutional unit (c) relative to the total constitutional units of polymer A1 is preferably 30 mol% or less, more preferably 20 mol% or less, further preferably 10 mol% or less. Particularly preferably, polymer A1 does not contain constitutional unit (c).

The proportion of constitutional unit (d) relative to the total constitutional units of polymer A1 is preferably 30 mol% or less, more preferably 20 mol% or less, further preferably 10 mol% or less. Particularly preferably, polymer A1 does not contain constitutional unit (d).

Among the polymers of the present invention, a copolymer containing constitutional unit (c) (excluding copolymer containing constitutional unit (b)) (e.g., copolymer containing constitutional unit (a), constitutional unit (c), and constitutional unit (d)) are classified as polymer A2, not polymer A3.

The proportion of constitutional unit (d) relative to the total constitutional units of polymer A2 is preferably 30 mol% or less, more preferably 20 mol% or less, further preferably 10 mol% or less. Particularly preferably, polymer A2 does not contain constitutional unit (d).

Polymers A1 to A3 may contain, as long as the effect of the present invention is not impaired, a constitutional unit (e) derived from other monomer different from monomer a to monomer d.

Examples of other monomer include C₁-C₆ alkyl (meth)acrylate, (meth)acrylic acid, isobornyl (meth)acrylate, and the like. Among these, C₁-C₆ alkyl (meth)acrylate is preferred, butyl (meth)acrylate is more preferred, and butyl methacrylate is further preferred.

Only one kind of other monomer may be used, or two or more kinds thereof may be used in combination. As other monomer, a commercially available product can be used, or it may be produced by a known method.

The proportion of constitutional unit (e) relative to the total constitutional units of polymer A1 is preferably 10 to 60 mol%, more preferably 20 to 50 mol%, further preferably 30 to 45 mol%.

The proportion of constitutional unit (e) relative to the total constitutional units of polymer A2 or A3 is preferably 30 mol% or less, more preferably 20 mol% or less, further preferably 10 mol% or less. Particularly preferably, polymer A2 and polymer A3 do not contain constitutional unit (e).

Polymer A1 is a copolymer preferably consisting of constitutional unit (a), constitutional unit (b), and constitutional unit (e). As used herein, the "copolymer consisting of constitutional unit (a), constitutional unit (b), and constitutional unit (e)" means a copolymer in which all of the constitutional units (repeat units) in the copolymer consist of constitutional unit (a), constitutional unit (b), and constitutional unit (e). Other terms similar to "copolymer consisting of constitutional unit (a), constitutional unit (b), and constitutional unit (e" also mean the same as the "copolymer consisting of constitutional unit (a), constitutional unit (b), and constitutional unit (e)".

Polymer A1 is more preferably a copolymer consisting of
one or two kinds of constitutional units (a) derived from 2-(meth)acryloyloxyethyl phosphorylcholine,
one or more kinds of constitutional units (b) derived from glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, or sorbitol mono(meth)acrylate, and
one or more kinds of constitutional units (e) derived from C₁-C₆ alkyl (meth) acrylate.

Polymer A1 is further preferably a copolymer consisting of
one or two kinds of constitutional units (a) derived from 2-(meth)acryloyloxyethyl phosphorylcholine,
one or two kinds of constitutional units (b) derived from glycerol mono(meth)acrylate, and
one or two kinds of constitutional units (e) derived from butyl (meth)acrylate.

Polymer A1 is particularly preferably a copolymer consisting of
constitutional unit (a) derived from 2-methacryloyloxyethyl phosphorylcholine,
constitutional unit (b) derived from glycerol monomethacrylate, and
constitutional unit (e) derived from butyl methacrylate.

Polymer A2 is preferably a copolymer consisting of constitutional unit (a) and constitutional unit (c).

Polymer A2 is more preferably a copolymer consisting of
one or two kinds of constitutional units (a) derived from 2-(meth)acryloyloxyethyl phosphorylcholine, and
one or more kinds constitutional units (c) derived from polyethylene glycol mono(meth)acrylate, methoxypolyethylene glycol (meth)acrylate, or ethoxypolyethylene glycol (meth)acrylate.

Polymer A2 is further preferably a copolymer consisting of
one or two kinds of constitutional units (a) derived from 2-(meth)acryloyloxyethyl phosphorylcholine, and
one or more kinds of constitutional units (c) derived from methoxypolyethylene glycol (meth)acrylate, or ethoxypolyethylene glycol (meth)acrylate.

Polymer A2 is particularly preferably a copolymer consisting of
constitutional unit (a) derived from 2-methacryloyloxyethyl phosphorylcholine, and
one or two kinds of constitutional units (c) derived from methoxypolyethylene glycol methacrylate or ethoxypolyethylene glycol methacrylate.

Polymer A2 is most preferably a copolymer consisting of
constitutional unit (a) derived from 2-methacryloyloxyethyl phosphorylcholine, and
constitutional unit (c) derived from methoxypolyethylene glycol methacrylate.

Polymer A3 is preferably a copolymer consisting of constitutional unit (a) and constitutional unit (d).

Polymer A3 is more preferably a copolymer consisting of
one or two kinds of constitutional units (a) derived from 2-(meth)acryloyloxyethyl phosphorylcholine, and
one or more kinds of constitutional units (d) derived from benzyl (meth)acrylate, 1-phenylethyl (meth)acrylate, 2-phenylethyl (meth)acrylate, or phenoxyethylene glycol (meth)acrylate.

Polymer A3 is further preferably a copolymer consisting of
one or two kinds of constitutional units (a) derived from 2-(meth)acryloyloxyethyl phosphorylcholine, and
one or more kinds of constitutional units (d) derived from benzyl (meth)acrylate, 1-phenylethyl (meth)acrylate, or 2-phenylethyl (meth)acrylate.

Polymer A3 is particularly preferably a copolymer consisting of
one or two kinds of constitutional units (a) derived from 2-(meth)acryloyloxyethyl phosphorylcholine, and
one or two kinds of constitutional units (d) derived from benzyl (meth)acrylate.

Polymer A3 is most preferably a copolymer consisting of
constitutional unit (a) derived from 2-methacryloyloxyethyl phosphorylcholine, and
constitutional unit (d) derived from benzyl methacrylate.

The polymer of the present invention may be a random copolymer, an alternating copolymer, a block copolymer, a graft polymer, or a copolymer containing two or more structures thereof. It is preferably a random copolymer from the viewpoint of the manufacturability of the polymer of the present invention.

The polymers A1 to A3 can be produced by known methods (e.g., the method described in WO 2018/216628).

The nucleic acid adsorption inhibitor of the present invention can contain components other than polymers A1 to A3, as long as the nucleic acid adsorption inhibitory effect of the polymers A1 to A3 is not impaired. Such other components are not particularly limited, and may be appropriately selected from, for example, those exemplified as "other components" in the nucleic acid solution of the present invention described below.

Polymers A1 to A3 contained in a single-stranded nucleic acid solution can be easily used as a single-stranded nucleic acid adsorption inhibitor.

Methods for containing the polymer of the present invention in a nucleic acid solution include adding polymers A1 to A3 to an already prepared single-stranded nucleic acid solution and dissolving them; dissolving the polymer of the present invention in advance in a solvent such as a buffer that dissolves single-stranded nucleic acids; or placing the polymer of the present invention in advance in a container in which a single-stranded nucleic acid solution will be prepared and then adding the single-stranded nucleic acid solution thereto to dissolve same.

The concentration (final concentration) of the nucleic acid adsorption inhibitor of the present invention to be added to a single-stranded nucleic acid solution is preferably 0.01 to 5 w/v%, more preferably 0.1 to 1 w/v%, further preferably 0.1 to 0.5 w/v%. When this amount added is too small, the single-stranded nucleic acid adsorption inhibitory effect may not be achieved, whereas when the amount added is too large, problems such as reaction inhibition may occur when the single-stranded nucleic acid solution is used in applications such as enzyme reactions.

The single-stranded nucleic acid to which the single-stranded nucleic acid adsorption inhibitor of the present invention can be applied may be either RNA or single-stranded DNA, preferably RNA.

Here, the single-stranded nucleic acid may be artificially synthesized by chemical synthesis, in vitro synthesis (e.g., reverse transcription reaction), PCR, or the like, or may be prepared from cells, microorganisms, viruses, or the like by known methods. Here, the cells, microorganisms, viruses, and the like may be those collected from human, animals, or plants in the natural world or environment, or may be those obtained by isolation and culture.

### [Nucleic acid solution]

The present invention further provides a nucleic acid aqueous solution containing the single-stranded nucleic acid adsorption inhibitor of the present invention and a single-stranded nucleic acid.

The concentration (final concentration) of the single-stranded nucleic acid adsorption inhibitor in the nucleic acid solution of the present invention is, as described above, preferably 0.01 to 5 w/v%, more preferably 0.1 to 1 w/v%, further preferably 0.1 to 0.5 w/v%.

The single-stranded nucleic acid contained in the nucleic acid solution of the present invention may be either RNA or single-stranded DNA, preferably RNA.

The single-stranded nucleic acid may be artificially synthesized (e.g., RNA, complementary DNA, and the like) by chemical synthesis, in vitro synthesis (e.g., reverse transcription reaction, solid phase synthesis, and the like), PCR, or the like, or may be provided as a virus, bacterial cell, cell, body fluid, tissue, etc., or a suspension of any of these, or a nucleic acid extract prepared from any of these. The virus, bacterial cell, cell, body fluid, tissue, etc. may be collected from human, animals, or plants in the natural world or environment, or may be obtained by isolation and culture. From another viewpoint, the single-stranded nucleic acid may be a single-stranded nucleic acid present in a living body, such as messenger RNA, transfer RNA, ribosomal RNA, non-coding RNA, microRNA, ribozyme, single-stranded genomic RNA, single-stranded genomic DNA, and the like, or may be a single-stranded nucleic acid having a primary structure that is equivalent to or complementary to the entirety or a part of the aforementioned single-stranded nucleic acid, or may be a single-stranded nucleic acid having a primary structure entirely artificially designed.

The concentration of the single-stranded nucleic acid is determined appropriately according to the application using the nucleic acid.

The nucleic acid solution of the present invention may contain other components as long as the effects of the present invention are not impaired.

Examples of other component include polyol, polyether, protein, salts, buffer, surfactant, solvent, biochemical reagent, dyes, preservative, oil, solid support, and the like.

Examples of the polyol include glycerol, sucrose, glucose, and the like.

Examples of the polyether include polyoxyethylene glycol and the like.

Examples of the protein include albumin, gelatin, casein, enzyme, and the like.

Examples of the salts include alkali metal salts, alkaline earth metal salts, salts of amino acids, salts of peptides, salts of organic acids such as ethylenediaminetetraacetic acid and the like, and the like.

Examples of the buffer include Tris-HCl buffer, Good's buffer, glycine buffer, borate buffer, TE buffer, TAE buffer, TBE buffer, SSC buffer, and the like.

Examples of the surfactant include polyoxyethylene alkyl ether, sorbitan polyoxyethylene monoalkyl ether, alkyl betaine, and the like.

Examples of the solvent include water and organic solvents. Examples of the organic solvent include ethanol, propanol, isoamyl alcohol, glycerol, dimethylformamide, dimethyl sulfoxide, tetrahydrofuran, chloroform, phenol, and the like. The solvent is preferably water, and the nucleic acid solution of the present invention is preferably an aqueous nucleic acid solution. The nucleic acid aqueous solution may further contain an organic solvent.

Examples of the biochemical reagent include flavins and the like.

Examples of the dye include nucleic acid staining reagents such as ethidium bromide, SYBR^{™} Green I, and the like, fluorescent dyes such as ROX^{™} Dye and the like, colorants such as Orange G, bromophenol blue, xylene cyanol FF and the like, and the like.

Examples of the preservative include sodium azide, parahydroxybenzoic acid preparation, dehydroacetic acid preparation, Proclin preparation, and the like.

Examples of the oil include mineral oil and the like.

Examples of the solid support include silica beads, magnetic beads, and the like.

### [Nucleic acid amplification method]

The nucleic acid solution of the present invention can be subjected to an enzyme reaction while still containing the single-stranded nucleic acid adsorption inhibitor of the present invention.

Examples of the enzyme reaction include cleavage by nuclease, reverse transcription by reverse transcriptase, replication by DNA polymerase, nucleic acid amplification applying these, and the like. The enzyme reaction in the present invention is preferably replication of single-stranded DNA or RNA, more preferably reverse transcription PCR.

### [Method for inhibiting adsorption of single-stranded nucleic acid]

The present invention provides a method for inhibiting adsorption of a single-stranded nucleic acid in a nucleic acid solution to a member in contact with the aforementioned nucleic acid solution, including mixing the single-stranded nucleic acid adsorption inhibitor of the present invention, the single-stranded nucleic acid, and a solvent. The aforementioned mixing is not particularly limited. For example, (1) the single-stranded nucleic acid adsorption inhibitor of the present invention, a single-stranded nucleic acid, and a solvent may be mixed; (2) a solution containing a single-stranded nucleic acid and a solvent, and the single-stranded nucleic acid adsorption inhibitor of the present invention may be mixed; or (3) a solution containing the single-stranded nucleic acid adsorption inhibitor of the present invention and a solvent, and a single-stranded nucleic acid may be mixed. The explanations of the nucleic acid solution obtained by mixing the single-stranded nucleic acid adsorption inhibitor of the present invention, a single-stranded nucleic acid, and a solvent (e.g., the kind of single-stranded nucleic acid used, the concentration of the single-stranded nucleic acid adsorption inhibitor of the present invention, and other components) are the same as those of the aforementioned nucleic acid solution of the present invention.

### [Example]

The present invention is explained in more detail in the following by referring to Examples and the like, which are not to be construed as limitative.

### [Synthesis of polymer]

Polymer A1-1 to polymer A3-1, which are within the scope of the present invention, and polymer Z-1, which is outside the scope of the present invention, were prepared as follows. The obtained polymers were dissolved in Water, Nuclease free (manufactured by Nippon Gene Co., Ltd., hereafter referred to as "PW") to a concentration 10 times the final concentration described for each condition in the Examples and Comparative Examples described below, and the obtained polymer aqueous solutions were used.

The five kinds of polymers prepared are summarized in Table 1.

### [Synthetic Example 1]

Polymer A1-1 corresponding to polymer A1 was prepared as follows.

2-Methacryloyloxyethyl phosphorylcholine (hereinafter to be referred to as "MPC") (57 g), glycerol monomethacrylate (hereinafter to be referred to as "GLM") (15 g), and butyl methacrylate (hereinafter to be referred to as "BMA") (28 g) (MPC/GLM/BMA=40/20/40 (molar ratio)) were weighed in a glass flask for polymerization, and dissolved by adding ethanol (410 g) and purified water (200 g). This solution was heated to 60°C, 2,2'-azobis(isobutyronitrile) (hereinafter to be referred to as "AIBN") (1.9 g) was added under a nitrogen atmosphere, and the mixture was stirred for 5 hr to perform polymerization. The obtained polymerization solution was purified by dialysis using a semipermeable membrane with a molecular cutoff of 3,000, and then freeze-dried to obtain polymer A1-1. The weight average molecular weight of polymer A1-1 was 21,000 based on polyethylene glycol standard as measured by GPC under the below-mentioned conditions.

### [Synthetic Example 2]

Polymer A2-1 corresponding to polymer A2 was prepared as follows.

MPC (84 g) and methoxypolyethylene glycol methacrylate (number average molecular weight of polyethylene glycol chain: about 500, hereinafter to be referred to as "PEG-MA") (16 g) (MPC/PEG-MA=90/10 (molar ratio)) were weighed in a glass flask for polymerization, and dissolved by adding purified water (233 g). To the obtained solution was added 4,4'-azobis(4-cyanovaleric acid) (2.6 g). The solution was heated to 70°C and stirred under a nitrogen atmosphere for 6 hr to perform polymerization. The obtained polymerization solution was purified by dialysis using a semipermeable membrane with a molecular cutoff of 20,000, and then freeze-dried to obtain polymer A2-1. The weight average molecular weight of polymer A2-1 was 133,000 based on polyethylene glycol standard as measured by GPC under the below-mentioned conditions.

### [Synthetic Example 3]

Polymer A3-1 corresponding to polymer A3 was prepared as follows.

MPC (87 g) and benzyl methacrylate (hereinafter to be referred to as "BzMA") (13 g) (MPC/BzMA=80/20 (molar ratio)) were weighed in a glass flask for polymerization, and dissolved by adding ethanol (hereinafter to be referred to as "EtOH") (233 g). This solution was heated to 60°C, AIBN (0.33 g) was added under a nitrogen atmosphere, and the mixture was stirred for 6 hr to perform polymerization. The obtained polymerization solution was purified by dialysis using a semipermeable membrane with a molecular cutoff of 3,000, and then freeze-dried to obtain polymer A3-1. The weight average molecular weight of polymer A3-1 was 240,000 based on polyethylene glycol standard as measured by GPC under the below-mentioned conditions.

### [Synthetic Example 4]

Polymer Z-1 which is outside the scope of the present invention was prepared as follows.

MPC (47 g) and BMA (53 g) (MPC/BMA=30/70 (molar ratio)) were weighed in a glass flask for polymerization, and dissolved by adding EtOH (900 g). This solution was heated to 60°C, AIBN (2.4 g) was added under a nitrogen atmosphere, and the mixture was stirred for 7 hr to perform polymerization. The obtained polymerization solution was purified by dialysis using a semipermeable membrane with a molecular cutoff of 3,000, and then freeze-dried to obtain polymer Z-1. The weight average molecular weight of polymer Z-1 was 94,000 based on polyethylene glycol standard as measured by GPC under the below-mentioned conditions.

### [GPC measurement]

The GPC measurement of each polymer obtained in Synthetic Examples 1 to 4 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

**[Table 1]**

| | monomer a | | monomers b to d | | | other monomer | | weight average molecular weight [×10³] |
|---|---|---|---|---|---|---|---|---|
| | kind | proportion [mol%] | classing | kind | proportion [mol%] | kind | proportion [mol%] | |
| polymer A1-1 | MPC | 40 | monomer b | GLM | 20 | BMA | 40 | 21 |
| polymer A2-1 | MPC | 90 | monomer c | PEG-MA | 10 | - | - | 133 |
| polymer A3-1 | MPC | 80 | monomer d | BZMA | 20 | - | - | 240 |
| polymer Z-1 | MPC | 30 | - | - | - | BMA | 70 | 94 |

### [Experimental Example 1]

Polymer A1-1 to polymer A3-1 and polymer Z-1 obtained in Synthetic Examples 1 to 4 were each added to RNA solution. This was placed in a glass tube, an operation of placing the total amount in a fresh glass tube was repeated, and the solution was developed by electrophoresis. The residual ratio of RNA in the solution was compared by comparing the quantitative value of RNA with the control. That is, the amount of DNA remaining without adsorbing to the inner surface of the glass tube and a micro chip and the like that handled the solution was compared. In detail, the following experiments were performed.
1) RNA Ladder (manufactured by Nippon Gene Co., Ltd.) was used as RNA and added to the below-mentioned RNA solution to a final concentration of 2.5 ng/µL.
2) As the 10X buffer, 10X TE (manufactured by DOJINDO LABORATORIES) was used.
3) Each RNA solution listed in Table 2 was prepared in DNA LoBind Tube (manufactured by Eppendorf).
4) Entire amount of each of the solutions in 3) was transferred to LABORAN screw tube bottle No. 2 (manufactured by AS ONE Corporation, hereinafter to be referred to as "glass tube").
5) Entire amount of each of the solutions in 4) was transferred to a new glass tube.
6) Entire amount of each of the solutions in 5) was transferred to a new glass tube.
7) Entire amount of each of the solutions in 6) was transferred to a new glass tube.
8) Entire amount of each of the solutions in 7) was transferred to a new glass tube.
9) 50X TAE (manufactured by Nippon Gene Co., Ltd.) was diluted according to the manufacturer's instructions to prepare an electrophoresis buffer (1X TAE). 1X TAE was added in a proportion of 100 mL relative to 1 g of Agarose S (manufactured by Nippon Gene Co., Ltd.), boiled to dissolve same, and solidified to prepare an electrophoresis gel.
10) 7.5 µL of each of the solutions in 8) was removed and transferred to DNA LoBind Tube.
11) 7.5 µL of 2X RNA loading buffer (ethidium bromide-free) (manufactured by FUJIFILM Wako Pure Chemical Corporation) was added to each of the solutions in 10).
12) Each of the solutions in 11) was heated in a 70°C water bath for 10 min, then immediately cooled on ice for at least 1 min to prepare an electrophoresis sample.
13) The sample from 12) was developed by agarose gel electrophoresis. The electrophoresis buffer and gel from 9) were used, and 5 µL of sample was applied. Electrophoresis was performed at 100 V for about 30 min.
14) The gel was stained by immersing same for about 15 min in Midori Green Advance (manufactured by Nihon Genetics Co., Ltd.) diluted 10,000-fold with 1X TAE, followed by destaining in pure water for about 15 min.
15) Images were taken using a FASdigi Compact (manufactured by Nihon Genetics Co., Ltd.) under BlueGreen LED illumination. Quantitative values for the 6 kbase band were calculated using ImageJ (National Institutes of Health, USA). The quantitative values are shown as relative values with the control as 100. The results are summarized in Table 2.

**[Table 2]**

| | | | control 1 | Example 1-1 | Example 1-2 | Example 1-3 | Comparative Example 1-1 |
|---|---|---|---|---|---|---|---|
| composition | 10X buffer | | 5 µL | | | | |
| | RNA Ladder | | 2.5 µL (final concentration 2.5 ng/µL) | | | | |
| | polymer | kind | - | polymer A1-1 | polymer A2-1 | polymer A3-1 | polymer Z-1 |
| | | amount added (*1) | - | 5 µL | 5 µL | 5 µL | 5 µL |
| | PW | | rest | | | | |
| | total amount | | 50 µL | | | | |
| container | | | glass tube | | | | |
| results | quantitative value (*2) | | 100 | 140 | 129 | 170 | 39 |

| | | | | | | | |
|---|---|---|---|---|---|---|---|
| *1 All polymers were added to a final concentration of 0.1%(w/v). *2 Quantitative value is shown as relative value with the control as 100. | | | | | | | |

In Example 1-1 to Example 1-3 and Comparative Example 1-1, polymer A1-1 to polymer A3-1 and polymer Z-1 were respectively used as single-stranded nucleic acid adsorption inhibitors. From these results, it is clear that the use of the polymer of the present invention as a single-stranded nucleic acid adsorption inhibitor inhibits RNA adsorption to glass tubes and microchips, thereby increasing the amount of remaining RNA.

### [Experimental Example 2]

An experiment similar to Experimental Example 1 was performed using 2 mL polypropylene screw-cap tube (free-standing type) (manufactured by AS ONE Corporation, hereinafter to be referred to as "PP tube") instead of PP tube (manufactured by AS ONE, hereafter referred to as "glass tube").
1) Each RNA aqueous solution shown in Table 3 was prepared and dispensed into a PP tube.
2) Other conditions and procedures were the same as in Experimental Example 1. The results are shown in Table 3.

**[Table 3]**

| | | | control 2 | Example 2-1 | Example 2-2 | Example 2-3 | Comparative Example 2-1 |
|---|---|---|---|---|---|---|---|
| composition | 10X buffer | | 5 µL | | | | |
| | RNA Ladder | | 2.5 µL (final concentration 2.5 ng/µL) | | | | |
| | polymer | kind | - | polymer A1-1 | polymer A2-1 | polymer A3-1 | polymer Z-1 |
| | | amount added (*1) | - | 5 µL | 5 µL | 5 µL | 5 µL |
| | PW | | rest | | | | |
| | total amount | | 50 µL | | | | |
| container | | | PP tube | | | | |
| results | quantitative value (*2) | | 100 | 122 | 135 | 125 | 92 |

| | | | | | | | |
|---|---|---|---|---|---|---|---|
| *1 All polymers were added to a final concentration of 0.1%(w/v). *2 Quantitative value is shown as relative value with the control as 100. | | | | | | | |

In Example 2-1 to Example 2-3 and Comparative Example 2-1, experiments similar to Example 1-1 to Example 1-3 and Comparative Example 1-1 were respectively performed using PP tube instead of glass tube. From these results, it is clear that the use of the polymer of the present invention as a single-stranded nucleic acid adsorption inhibitor can inhibit single-stranded nucleic acid adsorption even when the container material is polypropylene.

### [Industrial Applicability]

By simply adding the single-stranded nucleic acid adsorption inhibitor of the present invention to a single-stranded nucleic acid solution, adsorption of the nucleic acid to members such as containers, microchips, and the like can be inhibited. As a result, reduction of loss due to adsorption to containers and the like during nucleic acid extraction, namely, improvement of recovery rates, can be expected and improvement of detection sensitivity can be expected, in, for example, genetic testing in the medical science, veterinary medicine, and forensic medical fields. Furthermore, improvement of storage stability and transportation stability can be expected by inhibiting the reduction in effective concentration due to adsorption to containers and the like during storage and transportation of single-stranded nucleic acid-containing specimens for genetic testing. In addition, the improvement of stability during transportation and storage of pharmaceutical products and the like containing single-stranded nucleic acids can be expected.

This application is based on a patent application No. 2023-074988 filed in Japan, the contents of which are encompassed in full herein.

## Claims

1. A single-stranded nucleic acid adsorption inhibitor comprising one or more polymers selected from the following Group A:
[Group A]
polymer A1: a copolymer comprising a constitutional unit (a) derived from 2-(meth)acryloyloxyethyl phosphorylcholine, and a constitutional unit (b) derived from C₂-C₆ alkyl (meth)acrylate having two or more hydroxy groups as substituents;
polymer A2: a copolymer comprising the constitutional unit (a), and a constitutional unit (c) derived from polyethylene glycol (meth)acrylate; and
polymer A3: a copolymer comprising the constitutional unit (a), and a constitutional unit (d) derived from C₁-C₆ alkyl (meth)acrylate having a phenyl group or a phenoxy group as a substituent.

2. A nucleic acid solution comprising the single-stranded nucleic acid adsorption inhibitor according to claim 1 and a single-stranded nucleic acid.

3. A method for amplifying a nucleic acid, comprising using the nucleic acid solution according to claim 2.
